# EUROPÄISCHE PATENTANMELDUNG

(11) **EP 3 282 271 A1**
(43) Veröffentlichungstag der Anmeldung: **14.02.2018**
(21) Anmeldenummer: 16183218.3
(22) Anmeldetag: 08.08.2016
(51) Int. Cl.: G01R 33/54, G06N 3/02

(54) **VERFAHREN ZU EINEM EINSTELLEN UND/ODER ANPASSEN VON EINEM PARAMETERWERT VON ZUMINDEST EINEM PARAMETER EINES MAGNETRESONANZPROTOKOLLS FÜR ZUMINDEST EINE MAGNETRESONANZSEQUENZ**

(71) Anmelder: Siemens Healthcare GmbH, 91052 Erlangen (DE)
(72) Erfinder: Kröll, Maria, 91052 Erlangen (DE)

(57) **Zusammenfassung**

Die Erfindung geht aus von einem Verfahren zu einem Einstellen und/oder Anpassen von einem Parameterwert von zumindest einem Parameter einer Magnetresonanzprotokolls für zumindest eine Magnetresonanzsequenz mit den folgenden Verfahrensschritten:
- Auswahl des Magnetresonanzprotokolls,
- Bereitstellen von Rahmenbedingungen für die Einstellung und/oder Anpassung des Parameterwerts des zumindest einen Parameters des Magnetresonanzprotokolls,
- Bereitstellen von Einstellungskriterien von vorhergehenden Einstellungen und/oder Anpassungen des Parameterwerts des zumindest einen Parameters des Magnetresonanzprotokolls und
- Einstellen und/oder Anpassen des Parameterwerts des zumindest einen Parameters des Magnetresonanzprotokolls in Abhängigkeit von den bereitgestellten Rahmenbedingungen und in Abhängigkeit von den bereitgestellten Einstellungskriterien.

## Beschreibung

Die vorliegende Erfindung betrifft ein Verfahren zu einem Einstellen und/oder Anpassen von einem Parameterwert zumindest eines Parameters eines Magnetresonanzprotokolls für zumindest eine Magnetresonanzsequenz. In Abhängigkeit von bereitgestellten Rahmenbedingungen und in Abhängigkeit von bereitgestellten Einstellungskriterien erfolgt ein Einstellen und/oder Anpassen des Parameterwerts des zumindest einen Parameters des Magnetresonanzprotokolls.

Um Magnetresonanzbilder eines Patienten zu erzeugen, müssen zunächst Magnetresonanzprotokolle von einem medizinischen Bedienpersonal ausgewählt werden. Diese Magnetresonanzprotokolle weisen eine Vielzahl an Parametern auf, von denen einzelne oder auch mehrere Parameter von dem medizinischen Bedienpersonal eingestellt und/oder angepasst werden müssen. Aufgrund der Vielzahl an einstellbaren und/oder anpassbaren Parametern ist es für das medizinische Bedienpersonal, insbesondere für ein in der Einstellung und/oder Anpassung von Parametern ungeübtes medizinisches Bedienpersonal, oft schwierig, eine korrekte Parameterauswahl zu treffen und/oder einen korrekten Parameterwert für eine erfolgreiche Magnetresonanzuntersuchung einzustellen. Zudem ist für einen Benutzer oft schwierig zu erkennen, welche Parameter innerhalb einer Magnetresonanzsequenz miteinander korrelieren, so dass bei einer Änderung eines Parameters weitere Parameter zwangsläufig eine Änderung erfahren. Diese Korrelation zwischen einzelnen Parametern erschwert zudem die Einstellung und/oder Anpassung der Parameterwerte innerhalb eines Magnetresonanzprotokolls für einen Benutzer.

Der vorliegenden Erfindung liegt insbesondere die Aufgabe zugrunde, eine einfache Anpassung und/oder Einstellung von Parameterwerten eines Magnetresonanzprotokolls einer Magnetresonanzsequenz für einen Benutzer zu ermöglichen. Die Aufgabe wird durch die Merkmale der unabhängigen Ansprüche gelöst. Vorteilhafte Ausgestaltungen sind in den Unteransprüchen beschrieben.

Die Erfindung geht aus von einem Verfahren zu einem Einstellen und/oder Anpassen von einem Parameterwert von zumindest einem Parameter einer Magnetresonanzprotokolls für zumindest eine Magnetresonanzsequenz mit den folgenden Verfahrensschritten:
- Auswahl des Magnetresonanzprotokolls,
- Bereitstellen von Rahmenbedingungen für die Einstellung und/oder Anpassung des Parameterwerts des zumindest einen Parameters des Magnetresonanzprotokolls,
- Bereitstellen von Einstellungskriterien von vorhergehenden Einstellungen und/oder Anpassungen des Parameterwerts des zumindest einen Parameters des Magnetresonanzprotokolls und
- Einstellen und/oder Anpassen des Parameterwerts des zumindest einen Parameters des Magnetresonanzprotokolls in Abhängigkeit von den bereitgestellten Rahmenbedingungen und in Abhängigkeit von den bereitgestellten Einstellungskriterien.

In diesem Zusammenhang soll unter einem Parameter insbesondere ein protokollspezifischer Parameter verstanden werden, der vorzugsweise von einem Benutzer vor einer Magnetresonanzuntersuchung an einem Patienten angepasst und/oder eingestellt werden kann. Der protokollspezifische Parameter kann beispielsweise eine Echozeit und/oder eine Schichtdicke und/oder eine Schichtorientierung usw. umfassen. Beispielsweise kann ein Benutzer durch eine geschickte Auswahl und/oder Anpassung der einzelnen Parameter eines Magnetresonanzprotokolls eine Bildqualität beeinflussen.

Unter einer Magnetresonanzsequenz soll dabei ein definierter Ablauf von einzelnen Gradientenpulsen und/oder Hochfrequenzpulsen verstanden werden. Innerhalb des Magnetresonanzprotokolls sind typischerweise für die Magnetresonanzsequenz Parameter enthalten, die einzelnen Kriterien für den Ablauf der Magnetresonanzsequenz festlegen.

Die Auswahl des Magnetresonanzprotokolls kann dabei von einem Benutzer, beispielsweise einem Arzt und/oder einem MTRA, erfolgen. Vorzugsweise erfolgt die Auswahl des Magnetresonanzprotokolls dabei mittels einer Benutzerschnittstelle, mittels der der Benutzer seine Auswahl tätigen kann. Die Benutzerstelle kann dabei von der Magnetresonanzvorrichtung umfasst sein oder eine mobile Benutzerstelle, wie beispielsweise ein mobiles Touch-Display und/oder Tablet-PC, umfassen, die mit einer Steuerungseinheit und/oder eines Servereinheit, die einen Zugriff auf die Magnetresonanzprotokolle ermöglichen, verbunden ist. Zudem kann es auch vorgesehen sein, dass die Auswahl des Magnetresonanzprotokolls anhand von bereitgestellten Patienteninformationen automatisch und/oder selbsttätig erfolgen kann, insbesondere mittels einer Steuerungseinheit.

Das Bereitstellen von Rahmenbedingungen für die Einstellung und/oder Anpassung des Parameterwerts erfolgt bevorzugt durch den Benutzer. Die Rahmenbedingungen können dabei zielorientierte Rahmenbedingungen, wie beispielsweise eine Messzeit und/oder eine Bildqualität und/oder eine Schichtdicke und/oder ein Kontrastverhältnis und/oder Signal/Rauschverhältnis usw., umfassen. Zur Einhaltung der Rahmenbedingungen, insbesondere der zielorientierten Rahmenbedingungen, ist es typischerweise erforderlich, einzelne Parameter anzupassen und/oder einzustellen. Des Weiteren ist es auch denkbar, dass die Rahmenbedingungen auch einen Wertebereich für den Parameterwert des zumindest einen Parameters umfassen. Das Bereitstellen der Rahmenbedingungen durch den Benutzer erfolgt mittels der Benutzerschnittstelle.

Die Rahmenbedingungen können zudem auch fixe Werte von Parametern umfassen, die zur Einhaltung der zielorientierten Rahmenbedingungen nicht geändert werden dürfen. Die fixen Werte von einzelnen Parametern können dabei bereits innerhalb des Magnetresonanzprotokolls hinterlegt und/oder gespeichert sein. Zudem ist auch eine Festlegung von einzelnen Parametern mit fixen Parameterwerten durch einen Benutzer mittels der Benutzerschnittstelle möglich.

Die Einstellungskriterien umfassen bevorzugt Informationen, die innerhalb eines Speichermediums und/oder einer Datenbank gespeichert sind und in dem Bereitstellungsschritt aus dem Speichermedium und/oder der Datenbank abgerufen werden können. Die Einstellungskriterien umfassen hierbei Informationen und/oder Einstellungen von Parameterwerten von bereits in der Vergangenheit ausgeführten und/oder angewandten Magnetresonanzsequenzen. Zudem können die Einstellungskriterien auch Bewertungsinformationen zu den eingestellten Parameterwerten umfassen, wobei die Bewertungsinformationen von einem Benutzer hinterlegt wurden. Das Bereitstellen von Einstellungskriterien wird bevorzugt mittels einer Steuerungseinheit ausgeführt, die hierzu auf das Speichermedium und/oder die Datenbank zugreifen kann.

Die Verfahrensschritte des Bereitstellens von Rahmenbedingungen für die Einstellung und/oder Anpassung des Parameterwerts und des Bereitstellens von Einstellungskriterien können in zeitlicher Hinsicht auch zeitgleich durchgeführt werden oder in einer wie immer gearteten Reihenfolge durchgeführt werden.

Der Verfahrensschritt des Einstellens und/oder des Anpassens des Parameterwerts des zumindest einen Parameters des Magnetresonanzprotokolls in Abhängigkeit von den bereitgestellten Rahmenbedingungen und von den bereitgestellten Einstellungskriterien wird bevorzugt automatisch und/oder selbsttätig mittels der Steuerungseinheit und/oder einer Recheneinheit durchgeführt. Die Steuerungseinheit und/oder die Recheneinheit weist hierzu vorzugsweise einen Prozessor auf. Zudem weist die Steuerungseinheit und/oder Recheneinheit erforderliche Computerprogramme und/oder eine erforderliche Software auf.

Die Steuerungseinheit und/oder Recheneinheit kann hierbei eine zentrale Steuerungseinheit und/oder eine zentrale Recheneinheit umfassen, die bevorzugt gespeicherte Einstellungskriterien von Magnetresonanzprotokollen, die an unterschiedlichen Magnetresonanzvorrichtungen ausgeführt werden, vorliegen können. Alternativ oder zusätzlich kann die Steuerungseinheit auch innerhalb einer Magnetresonanzvorrichtung angeordnet sein, so dass nur die gespeicherte Einstellungskriterien, die an dieser Magnetresonanzvorrichtung erfasst wurden, eingehen können.

Durch die erfindungsgemäße Ausgestaltung kann vorteilhaft eine automatisierte Einstellung und/oder Anpassung der Parameterwerte für ein Magnetresonanzprotokoll erreicht werden und damit eine einfache und zeitsparende Anpassung und/oder Einstellung von Parametern für einen Benutzer bereitgestellt werden. Die Einstellung und/oder Anpassung der Parameterwerte erfordert von dem Benutzer hierbei nur noch die Auswahl der Magnetresonanzsequenz und eine zumindest teilweise Festlegung und/oder Bereitstellung der Rahmenbedingungen. Dies ermöglicht insbesondere ungeübten und/oder unerfahrenen Benutzern die eine optimale Nutzung einzelner Magnetresonanzsequenzen, so dass eine Qualität der erfassten Magnetresonanzdaten erhöht werden kann. Des Weiteren können zur Einstellung und/oder Anpassung des Parameterwerts des zumindest einen Parameters automatisiert alle bisher verwendeten und zur Verfügung stehenden Einstellungskriterien genutzt werden, so dass auch eine Fehleranfälligkeit bei der Einstellung und/oder Anpassung der Parameterwerte des Magnetresonanzprotokolls verringert wird. Hierdurch kann zudem eine hohe Qualität der erfassten Bilddaten erreicht werden.

Besonders vorteilhaft erfolgt das Einstellen und/oder Anpassen des Parameterwerts des zumindest einen Parameters des Magnetresonanzprotokolls automatisch und/oder selbsttätig mittels einer Steuerungseinheit, wobei die Steuerungseinheit einen selbstlernenden Algorithmus aufweist, der die Parameterwerte anhand der Einstellungskriterien und/oder Rahmenbedingungen anpasst und/oder einstellt. Hierbei kann in Abhängigkeit der Rahmenbedingungen eine optimale Einstellung und/oder Anpassung, insbesondere eine individuelle und/oder eine situationsabhängige Einstellung und/oder Anpassung, der Parameterwerte für einzelne Parameter automatisiert erfolgen. Zudem können mittels des selbstlernenden Algorithmus Wiederholungen von Fehleinstellungen und/oder Fehlanpassungen von Parameterwerten vorteilhaft verhindert. Weiterhin kann eine Einstellung und/oder eine Anpassung der Protokollparameter der Parameter des Magnetresonanzprotokolls stetig, insbesondere mit jedem Einstellung und/oder Anpassung und anschließender Bewertung der Ergebnisse, verbessert werden.

Der selbstlernende Algorithmus basiert typischerweise auf einem maschinellen Lernen, bei dem Wissen aus Erfahrung generiert wird. Das maschinelle Lernen erfolgt mittels künstlicher neuronaler Netze. Mittels des maschinellen Lernens kann der selbstlernende Algorithmus Muster und Gesetzmäßigkeiten in Lerndaten, insbesondere in den gespeicherten eingestellten und/oder angepassten Parameterwerten und deren Bewertung dazu, erkennen. Der selbstlernende Algorithmus kann dabei aus Beispielen lernen und diese nach Beendigung der Lernphase verallgemeinern.

Insbesondere basiert der selbstlernende Algorithmus und/oder das maschinelle Lernen auf einem Deep-Learning-Verfahren, bei dem Wissen aus Erfahrung generiert wird. Beim Deep-Learning-Verfahren werden künstliche neuronale Netze vorzugsweise zu Ebenen angeordnet, die immer komplexere Merkmale verwenden, um beispielsweise den Inhalt von Bilddaten und/oder Kontraste in Bilddaten zu erkennen. Dies ermöglicht beispielsweise, große Datenbestände in Kategorien einzuteilen.

Ferner sieht die Erfindung vor, dass der selbstlernende Algorithmus, insbesondere das maschinelle Lernen, wie beispielsweise das Deep-Learning-Verfahren, alle in dem Speichermedium und/oder der Datenbank hinterlegten Einstellungskriterien für eine Anpassung und/oder Einstellung des Parameterwerts des zumindest einen Parameters des Magnetresonanzprotokolls berücksichtigt. Hierdurch kann eine optimale Anpassung und/oder Einstellung des Parameterwerts erreicht werden, da aufgrund der umfangreichen Datenmenge statistische und/oder zufällige Fehler bei der Anpassung und/oder Einstellung des Parameterwerts reduziert und/oder ausgeschlossen werden können.

Des Weiteren sieht die Erfindung vor, dass die Rahmenbedingungen für die Einstellung und/oder Anpassung des Parameterwerts des zumindest einen Parameters des Magnetresonanzprotokolls einen Wertebereich für den einzustellenden und/oder anzupassenden Parameterwert umfassen. Hierbei soll unter einem Wertebereich insbesondere ein Bereich verstanden werden, der diejenigen Werte enthält, die einen Einstellbereich und/oder Anpassungsbereich für den Parameterwert des zumindest einen Parameters vorgehen. Der Wertebereich für den Parameterwert kann dabei manuell durch einen Benutzer vorgegeben werden. Zudem kann es auch sein, dass der Wertebereich für den Parameterwert in einer Datenbank hinterlegt ist, beispielsweise wenn der Wertebereich für den Parameterwert stets einen gleichen Bereich umfassen soll.

Hierdurch kann ein sinnvoller Wertebereich vorgegeben werden. Zudem können derart besonders vorteilhaft auf individuelle Vorgaben eines Benutzers bei der Anpassung und/oder Einstellung des Parameterwerts des zumindest einen Parameters zu berücksichtigt werden. Auch können derart vorteilhaft Untersuchungsvorgaben, beispielsweise in einem Krankenhaus, bei denen der Wertebereich stets eine gleiche Größe aufweist, berücksichtigt werden.

Ferner kann es vorgesehen sein, dass die Rahmenbedingungen für die Einstellung und/oder Anpassung des Parameterwerts des zumindest einen Parameters des Magnetresonanzprotokolls zumindest einen weiteren Parameterwert eines weiteren Parameters des Magnetresonanzprotokolls umfassen, wobei der weitere Parameterwert einen fixen Parameterwert umfasst. In diesem Zusammenhang soll unter einem fixen Parameterwert insbesondere ein Parameterwert eines Parameters eines Magnetresonanzprotokolls verstanden werden, dessen Wert unabhängig von Einstellungen und/oder Änderungen von weiteren Parameterwerten, insbesondere bei einer Änderung und/oder Einstellung des einzustellenden Parameterwerts, einen fixen Wert aufweist. Bei der Einstellung und/oder Anpassung des Parameterwerts werden häufig zur Einhaltung der Rahmenbedingungen weitere Parameterwerte mitgeändert, die mit dem einzustellenden und/oder anzupassenden Parameterwert korrelieren. Der fixe Parameterwert dagegen behält seinen Wert unverändert bei, unabhängig von den Rahmenbedingungen und/oder der Einstellung und/oder Anpassung des Parameterwerts. Eine Definierung und/oder eine Festlegung eines Parameterwerts als fixen Parameterwert kann dabei durch den Benutzer erfolgen. Hierdurch kann ein Benutzer vorteilhaft einzelne Parameterwerte von Parametern, die für jede Messung stets einen gleichen Wert umfassen sollen, definieren und/oder festlegen.

In einer vorteilhaften Weiterbildung der Erfindung kann es vorgesehen sein, dass die Rahmenbedingungen für die Einstellung und/oder Anpassung des Parameterwerts des zumindest einen Parameters des Magnetresonanzprotokolls zielorientierte Rahmenbedingungen umfassen. Hierbei sollen unter zielorientierten Rahmenbedingungen insbesondere Rahmenbedingungen verstanden werden, die ein Ziel und/oder Ergebnis der Magnetresonanzuntersuchung umfassen. Die zielorientierten Rahmenbedingungen können dabei eine Messzeit der Magnetresonanzsequenz und/oder eine Bildqualität der erfassten Bilddaten und/oder eine Schichtdicke und/oder ein Kontrastverhältnis und/oder ein Signal/Rauschverhältnis usw. umfassen. Hierdurch ist es lediglich erforderlich, dass ein Benutzer nur die zielorientierten und/oder aufgabenorientierten Rahmenbedingungen festlegt, wie beispielsweise eine T1-Kontrast Messung mit einer bestimmten Schichtdicke und einer definierten Bildabdeckung, und eine anschließende Anpassung und/oder Einstellung der Parameterwerte aller anzupassenden und/oder einzustellenden Parameter erfolgt automatisch und/oder selbsttätig mittels des selbstlernenden Algorithmus, insbesondere des maschinellen Lernens, wie beispielsweise mittels des Deep-Learning-Verfahrens.

Vorzugsweise sind die Rahmenbedingungen für die Einstellung und/oder Anpassung des Parameterwerts des zumindest einen Parameters des Magnetresonanzprotokolls des Magnetresonanzprotokolls benutzerabhängig und/oder institutionsabhängig. Hierdurch kann eine individuelle Anpassung der einzustellenden und/oder anzupassenden Parameterwerte erreicht werden. Unter benutzerabhängigen Rahmenbedingungen sollen hierbei insbesondere Rahmenbedingungen verstanden werden, die von einem Benutzer festgelegt werden. Unter institutionsabhängigen Rahmenbedingungen sollen hierbei insbesondere Rahmenbedingungen verstanden werden, die einheitlich für eine Institution, wie beispielweise einheitlich für ein Krankenhaus, festgelegt werden.

Ferner kann es vorgesehen sein, dass die Einstellungskriterien bereits erfolgte Einstellungen und/oder Anpassungen des Parameterwerts des zumindest einen Parameters des Magnetresonanzprotokolls und eine Bewertung der bereits erfolgten Einstellungen und/oder Anpassungen des Parameterwerts des zumindest einen Parameters umfassen. Die Bewertung umfasst bevorzugt eine Information, wie gut und/oder erfolgreich die angepassten und/oder eingestellten Parameterwerte zu den vorher festgelegten Rahmenbedingungen übereinstimmen. Vorzugsweise wurden die Bewertungen von einem Benutzer hinterlegt. Durch diese Ausgestaltung der Erfindung kann mittels des selbstlernenden Algorithmus, insbesondere des maschinellen Lernens, wie beispielsweise des Deep-Learning-Verfahrens, die Einstellung und/oder Anpassung der Parameterwerte optimiert werden und damit eine Qualität der erfassten Bilddaten stetig verbessert werden. Zudem können hierdurch Interaktionen eines Benutzers während Einstellen und/oder Anpassen der Parameterwerte, beispielsweise einem Korrigieren der von dem selbstlernenden Algorithmus, insbesondere dem maschinelle Lernen, wie beispielsweise dem Deep-Learning-Verfahren, eingestellten und/oder angepassten Parameterwerte, reduziert und/oder auf diese Interaktionen gänzlich verzichtet werden.

Eine vorteilhafte Weiterbildung der Erfindung sieht vor, dass bei einem Überschreiten der Rahmenbedingungen und/oder von weiteren Zielvorgaben während der Anpassung und/oder Einstellung des Parameterwerts des zumindest einen Parameters des Magnetresonanzprotokolls automatisch eine alternative Anpassungsstrategie gewählt wird, bei der mehrere Parameterwerte von weiteren Parametern des Magnetresonanzprotokolls angepasst und/oder eingestellt werden. Die weiteren Zielvorgaben können insbesondere Sicherheitsvorgaben und/oder patientenspezifischen Zielvorgaben usw. umfassen. Beispielsweise kann eine weitere Zielvorgabe einen Wert für eine spezifische Absorptionsrate (SAR) umfassen, der während einer Magnetresonanzuntersuchung nicht überschritten werden darf. Die alternative Anpassungsstrategie umfasst bevorzugt eine Anpassung und/oder Einstellung von Parameterwerten von mehr als einen Parameter, wobei die Einstellung von Parameterwerten der mehreren Parameter zu einer Einhaltung der Rahmenbedingungen und/oder der weiteren Zielvorgaben führt. Zudem kann auch die alternative Anpassungsstrategie eine Anpassung der Rahmenbedingungen umfassen.

Hierdurch kann vorteilhaft eine automatische Anpassung und/oder Korrektur der einstellbaren und/oder anpassbaren Parameterwerte und/oder der Rahmenbedingungen erfolgen, ohne dass hierbei eine Benutzeraktion erforderlich ist. Dies ermöglicht eine besonders einfache und schnelle Korrektur und/oder Anpassung der Parameterwerte und/oder der Rahmenbedingungen. Die Auswahl der Anpassungsstrategie wird bevorzugt mittels der Steuerungseinheit, insbesondere mittels des selbstlernenden Algorithmus und/oder maschinellen Lernens, wie beispielsweise des Deep-Learning-Verfahrens, der Steuerungseinheit, ausgewählt und durchgeführt.

Besonders vorteilhaft wird die Anpassungsstrategie situationsabhängig ausgewählt. Vorzugsweise erfolgt die situationsabhängige Auswahl der Anpassungsstrategie mittels der Steuerungseinheit, insbesondere mittels des selbstlernenden Algorithmus und/oder des maschinelle Lernens, wie beispielsweise des Deep-Learning-Verfahrens, der Steuerungseinheit. Dies ermöglicht eine besonders individuelle, die aktuelle Situation berücksichtigende Korrektur und/oder Anpassung der einzelnen Parameter bzw. der Rahmenbedingungen an die weiteren Zielvorgaben.

Des Weiteren kann es in einer weiteren Ausgestaltung der Erfindung vorgesehen sein, dass die Anpassungsstrategie diejenigen Parameterwerte von den weiteren Parametern des Magnetresonanzprotokolls anpasst und/oder ändert, die eine minimale Änderung der Rahmenbedingungen und/oder der Einstellungskriterien hervorrufen. Dies ermöglicht eine besonders individuelle Anpassung der einzelnen Parameter an die Zielvorgaben und an die Rahmenbedingungen und/oder an die Einstellungskriterien. Zudem kann die Magnetresonanzsequenz mit nur minimalen Abweichungen von einer idealen Einstellung ausgeführt werden.

Des Weiteren sieht die Erfindung vor, dass der eingestellte und/oder angepasste Parameterwert des zumindest einen Parameters des Magnetresonanzprotokolls gespeichert wird. Vorzugsweise wird hierbei der eingestellte und/oder angepasste Parameterwert der Datenbank mit den bereits getätigten Einstellungen und/oder Anpassungen des Parameterwerts hinzugefügt. Hierdurch kann eine ständige Erweiterung des zur Verfügung stehenden Datensatzes mit bereits erfolgten Einstellungen und/oder Anpassungen des Parameterwerts erreicht werden und damit eine Anpassungsrisiko und/oder ein Einstellungsrisiko bei zukünftigen Einstellungen und/oder Anpassungen des Parameterwerts minimiert werden.

Ferner sieht die Erfindung vor, dass der eingestellte und/oder angepasste Parameterwert des zumindest einen Parameters des Magnetresonanzprotokolls bewertet wird und die Bewertung zusammen mit dem eingestellten und/oder angepassten Parameterwert des zumindest einen Parameters des Magnetresonanzprotokolls gespeichert wird. Vorzugsweise erfolgt die Bewertung des eingestellten und/oder angepassten Parameterwerts durch einen Benutzer mittels einer Benutzerschnittstelle. Die Bewertung wird bevorzugt erst nach einem Ausführen der Magnetresonanzsequenz durchgeführt. Mittels der Bewertung kann ein Benutzer angeben, wie gut die eingestellten und/oder angepassten Parameterwerte mit den Rahmenbedingungen und/oder seinen Erwartungen übereinstimmen. Hierdurch kann ebenfalls eine ständige Erweiterung des zur Verfügung stehenden Datensatzes mit bereits getätigten Einstellungen und/oder Anpassungen des Parameterwerts erreicht werden und damit eine Anpassungsrisiko und/oder ein Einstellungsrisiko bei zukünftigen Einstellungen und/oder Anpassungen des Parameterwerts minimiert werden. Insbesondere kann durch die Bewertung ein Datensatz bereitgestellt werden, der eine Erkennung von Mustern und Gesetzmäßigkeiten mittels des selbstlernenden Algorithmus, insbesondere des maschinellen Lernens, wie beispielsweise mittels des Deep-Learning-Verfahrens, erlaubt. Dies ermöglicht mittels des selbstlernenden Algorithmus, insbesondere des maschinellen Lernens, wie beispielsweise des Deep-Learning-Verfahrens, eine optimierte Einstellungsstrategie und/oder Anpassungsstrategie für die Einstellung und/oder Anpassung der Parameterwerte zur Verfügung zu stellen. Zudem kann die Einstellung und/oder Anpassung der Parameterwerte mittels des selbstlernenden Algorithmus, insbesondere des maschinellen Lernens, wie beispielsweise mittels des Deep-Learning-Verfahrens, individuelle und/oder situationsabhängigen angepasst werden.

Alternativ oder zusätzlich kann es auch vorgesehen sein, dass zusammen mit den einstellten und/oder angepassten Parameterwerten auch die erfassten Bilddaten in der Datenbank und/oder dem Speichermedium hinterlegt werden. Des ermöglicht auch den selbstlernenden Algorithmus, insbesondere dem maschinellen Lernen, wie beispielsweise dem Deep-Learning-Verfahren, in den erfassten Bilddaten Muster und/oder Gesetzmäßigkeiten zu erkennen und daraus eine Einstellungsstrategie und/oder eine Anpassungsstrategie für die Einstellung und/oder Anpassung der Parameterwerte abzuleiten.

Des Weiteren geht die Erfindung aus von einer Magnetresonanzvorrichtung mit einer Steuerungseinheit und einer Benutzerschnittstelle, wobei die Magnetresonanzvorrichtung zu einer Durchführung eines Verfahrens zu einem Einstellen und/oder Anpassen von einem Parameterwert zumindest eines Parameter eines Magnetresonanzprotokolls für zumindest eine Magnetresonanzsequenz ausgelegt ist.

Es kann vorteilhaft eine automatisierte Einstellung und/oder Anpassung der Parameterwerte für ein Magnetresonanzprotokoll erreicht werden und damit eine einfache und zeitsparende Anpassung und/oder Einstellung von Parametern für einen Benutzer bereitgestellt werden. Die Einstellung und/oder Anpassung der Parameterwerte erfordert von dem Benutzer hierbei nur noch die Auswahl der Magnetresonanzsequenz und eine zumindest teilweise Festlegung und/oder Bereitstellung der Rahmenbedingungen. Dies ermöglicht insbesondere ungeübten und/oder unerfahrenen Benutzern die eine optimale Nutzung einzelner Magnetresonanzsequenzen, so dass eine Qualität der erfassten Magnetresonanzdaten erhöht werden kann. Des Weiteren können zur Einstellung und/oder Anpassung des Parameterwerts des zumindest einen Parameters automatisiert alle bisher verwendeten und zur Verfügung stehenden Einstellungskriterien genutzt werden, so dass auch eine Fehleranfälligkeit bei der Einstellung und/oder Anpassung der Parameterwerte des Magnetresonanzprotokolls verringert wird. Hierdurch kann zudem eine hohe Qualität der erfassten Bilddaten erreicht werden.

Die Vorteile der erfindungsgemäßen Magnetresonanzvorrichtung entsprechen im Wesentlichen den Vorteilen des erfindungsgemäßen Verfahrens zu einem Einstellen und/oder Anpassen von einem Parameterwert zumindest eines Parameters eines Magnetresonanzprotokolls für zumindest eine Magnetresonanzsequenz, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Des Weiteren geht die Erfindung aus von einem Computerprogrammprodukt, welches ein Programm umfasst und direkt in einem Speicher einer programmierbaren Steuerungseinheit ladbar ist, mit Programmmitteln, um ein Verfahren zu einem Einstellen und/oder Anpassen von zumindest einem Parameterwert eines Parameters eines Magnetresonanzprotokolls für zumindest eine Magnetresonanzsequenz auszuführen, wenn das Programm in der Steuerungseinheit ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch compiliert und gebunden oder der nur interpretiert werden muss, oder einen ausführbaren Softwarecode umfassen, der zur Ausführung nur noch in eine entsprechende Recheneinheit zu laden ist.

Des Weiteren geht die Erfindung aus von einem Computerlesbarer Datenträger, welcher ein Programm umfasst, das zu einer Ausführung eines Verfahrens zu einem Einstellen und/oder Anpassen von zumindest einem Parameterwert eines Parameters eines Magnetresonanzprotokolls für zumindest eine Magnetresonanzsequenz vorgesehen ist.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine Magnetresonanzvorrichtung in einer schematischen Darstellung und
- Fig. 2: ein erfindungsgemäßes Verfahren zu einem Einstellen und/oder Anpassen von einem Parameterwert zumindest eines Parameters eines Magnetresonanzprotokolls für zumindest eine Magnetresonanzsequenz.

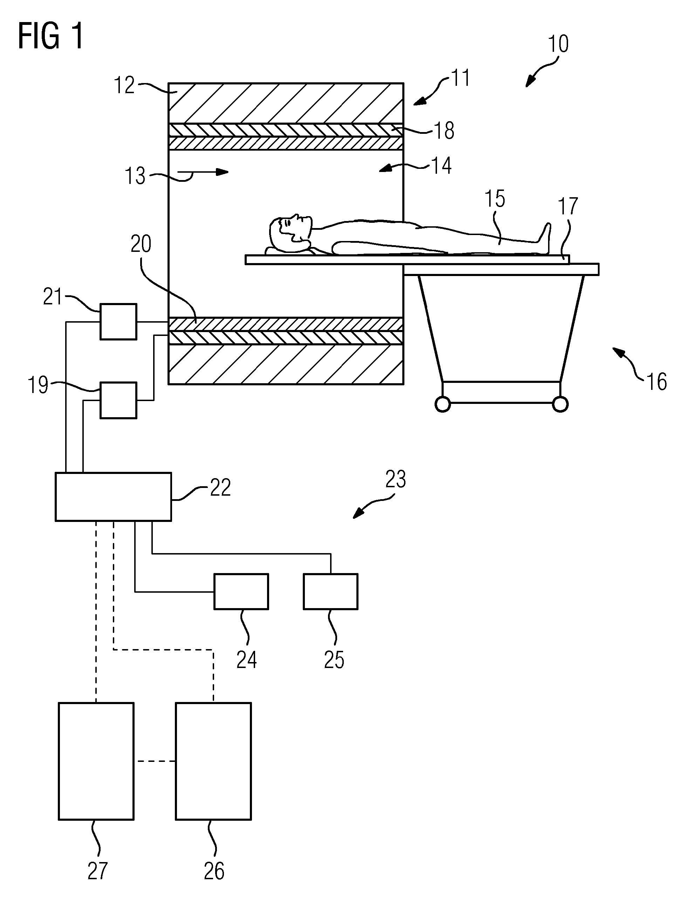

In Fig. 1 ist eine Magnetresonanzvorrichtung 10 schematisch dargestellt. Die Magnetresonanzvorrichtung 10 umfasst eine Magneteinheit 11, die einen supraleitenden Hauptmagneten 12 zu einem Erzeugen eines starken und insbesondere konstanten Hauptmagnetfelds 13 umfasst. Zudem weist die Magnetresonanzvorrichtung 10 einen Patientenaufnahmebereich 14 auf zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20 zu einer Anregung einer Polarisation, die sich in dem von dem Hauptmagneten 12 erzeugten Hauptmagnetfeld 13 einstellt. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in einen Untersuchungsraum, der im Wesentlichen von einem Patientenaufnahmebereich 14 der Magnetresonanzvorrichtung 10 gebildet ist, ein.

Zu einer Steuerung des Hauptmagneten 12, der Gradientensteuereinheit 18 und zur Steuerung der Hochfrequenzantennensteuereinheit 20 weist die Magnetresonanzvorrichtung 20 eine Steuerungseinheit 22 auf. Die Steuerungseinheit 22 steuert zentral die Magnetresonanzvorrichtung 10, wie beispielsweise das Durchführen einer vorbestimmten bildgebenden Gradientenechosequenz. Zudem umfasst die Steuerungseinheit 22 eine nicht näher dargestellte Auswerteeinheit zu einer Auswertung von medizinischen Bilddaten, die während der Magnetresonanzuntersuchung erfasst werden. Des Weiteren umfasst die Magnetresonanzvorrichtung 10 eine Benutzerschnittstelle 23, die mit der Steuerungseinheit 22 verbunden ist. Steuerinformationen wie beispielsweise Bildgebungsparameter, sowie rekonstruierte Magnetresonanzbilder können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle eine Eingabeeinheit 25 auf.

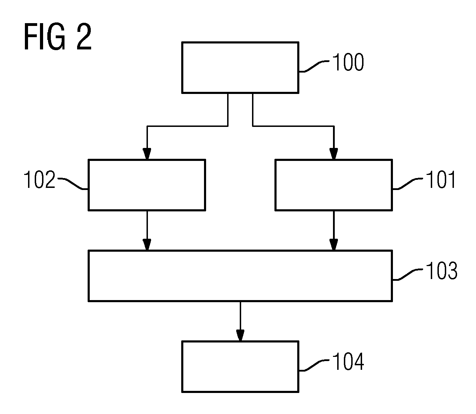

In Fig. 2 ist ein erfindungsgemäßes Verfahren zu einem Einstellen und/oder Anpassen von einem Parameterwert zumindest eines Parameters eines Magnetresonanzprotokolls für zumindest eine Magnetresonanzsequenz dargestellt. Zur Ausführung des Verfahrens zu einem Einstellen und/oder Anpassen von zumindest einem Parameterwert eines Parameters eines Magnetresonanzprotokolls für zumindest eine Magnetresonanzsequenz steht die Steuerungseinheit 22 und eine Benutzerschnittstelle 26 zur Verfügung.

Die Benutzerschnittstelle 26 ist vorliegend von einer separat zur Magnetresonanzvorrichtung 10 ausgebildeten Benutzerschnittstelle 26 umfasst, wobei die Benutzerschnittstelle 26 von einer mobilen Benutzerschnittstelle 26 umfasst. Die mobile Benutzerschnittstelle 26 kann dabei in einem mobilen Touch-Display und/oder einem Tablet-PC und/oder einem Mobiltelefon usw. integriert sein. Alternativ oder zusätzlich kann auch die Benutzerschnittstelle 23 der Magnetresonanzvorrichtung 10 für die Ausführung des Verfahrens zu einem Einstellen und/oder Anpassen von einem Parameterwert zumindest eines Parameters eines Magnetresonanzprotokolls für zumindest eine Magnetresonanzsequenz zur Verfügung stehen.

Zur Ausführung des Verfahrens zu einem Einstellen und/oder Anpassen von einem Parameterwert zumindest eines Parameters eines Magnetresonanzprotokolls für zumindest eine Magnetresonanzsequenz weist die Steuerungseinheit 22 weist hierzu Computerprogramme und/oder Software auf, die direkt in einer Speichereinheit 27 ladbar sind, mit Programmmitteln, um ein Verfahren zu einem Einstellen und/oder Anpassen von Messparametern für eine Messsequenz einer Magnetresonanzuntersuchung auszuführen, wenn die Computerprogramme und/oder Software in der Steuerungseinheit 22 ausgeführt werden. Die Steuerungseinheit 22 weist hierzu einen nicht näher dargestellten Prozessor auf, der zu einer Ausführung der Computerprogramme und/oder Software ausgelegt ist.

Die Speichereinheit 27 im vorliegenden Ausführungsbeispiel ist von einer zur Steuerungseinheit 22 externen Speichereinheit 27 umfasst. Die Speichereinheit kann von einer Cloud umfasst sein. Alternativ hierzu können die Computerprogramme und/oder Software auch auf einer innerhalb der Steuerungseinheit angeordneten Speichereinheit 27 gespeichert sein.

In einem ersten Verfahrensschritt 100 des Verfahrens erfolgt eine Auswahl des Magnetresonanzprotokolls. Die Auswahl kann manuell durch einen Benutzer, beispielsweise ein die Magnetresonanzuntersuchung betreuendes medizinisches Bedienpersonal, mittels der Benutzerschnittstelle 23, 26 erfolgen. Dabei kann die Auswahl mittels der innerhalb der Magnetresonanzvorrichtung 10 angeordneten Benutzerschnittstelle 23 als auch mittels der separat zur Magnetresonanzvorrichtung 10 ausgebildeten Benutzerschnittstelle 26 erfolgen. Zudem ist es auch denkbar, dass die Auswahl aufgrund von bereits in der Steuerungseinheit 22 vorliegenden Patienteninformationen automatisch und/oder selbsttätig mittels der Steuerungseinheit 22 erfolgt.

In einem daran anschließenden Verfahrensschritt 101 erfolgt ein Bereitstellen von Rahmenbedingungen für die Einstellung und/oder Anpassung des Parameterwerts des zumindest einen Parameters des Magnetresonanzprotokolls, wobei die Rahmenbedingungen von dem Benutzer mittels der Benutzerschnittstelle 23, 26 bereitgestellt werden. Die Rahmenbedingungen sind somit benutzerabhängig, insbesondere kann der Benutzer die Rahmenbedingungen zur Erzielung eines bestimmten Messergebnisses vorgeben. Zudem können die Rahmenbedingungen zumindest teilweise auch institutsabhängig sein, indem zumindest einzelne Rahmenbedingungen für jede Anwendung einer bestimmten Magnetresonanzsequenz innerhalb beispielsweise eines Krankenhauses stets die gleichen sind.

Die Rahmenbedingungen für die Einstellung und/oder Anpassung des Parameterwerts des zumindest einen Parameters des Magnetresonanzprotokolls können hierbei einen Wertebereich für den einzustellenden und/oder anzupassenden Parameterwert umfassen. Der Wertebereich gibt dabei einen Bereich vor, innerhalb dessen der Wert des eingestellten und/oder angepassten Parameterwerts liegen muss und/oder sollte.

Die Rahmenbedingungen für die Einstellung und/oder Anpassung des Parameterwerts des zumindest einen Parameters des Magnetresonanzprotokolls können zudem auch einen weiteren Parameterwert umfassen, wobei der weitere Parameterwert einen fixen Parameterwert umfasst. Dieser fixe Parameterwert ist vorzugsweise von dem Benutzer festgelegt. Der fixe Parameter umfasst einen konstanten Wert, der dabei unabhängig von Einstellungen und/oder Änderungen von weiteren Parameterwerten, insbesondere bei einer Änderung und/oder Einstellung des einzustellenden Parameterwerts ist.

Des Weiteren können die Rahmenbedingungen für die Einstellung und/oder Anpassung des Parameterwerts des zumindest einen Parameters des Magnetresonanzprotokolls auch zielorientierte Rahmenbedingungen aufweisen. Die zielorientierten Rahmenbedingungen können beispielsweise eine Messzeit der Magnetresonanzsequenz und/oder eine Bildqualität der erfassten Bilddaten und/oder eine Schichtdicke und/oder ein Kontrastverhältnis und/oder ein Signal/Rauschverhältnis usw. umfassen. Die vorangehende Auszählung stellt hierbei keine abschließende Aufzählung sondern nur eine beispielhafte Aufzählung der zielorientierten Rahmenbedingungen dar.

Beispielsweise kann ein Benutzer als zielorientierte Rahmenbedingungen für eine Magnetresonanzsequenz eine T1-Kontrast Messung mit einer bestimmten Schichtdicke und einer definierten Bildabdeckung vorgeben.

In einem weiteren Verfahrensschritt 102 erfolgt ein Bereitstellen von Einstellungskriterien von vorhergehenden Einstellungen und/oder Anpassungen des Parameterwerts des zumindest einen Parameters des Magnetresonanzprotokolls. Der Verfahrensschritt 101 des Bereitstellens von Rahmenbedingungen für die Einstellung und/oder Anpassung des Parameterwerts des zumindest einen Parameters des Magnetresonanzprotokolls und der Verfahrensschritt 102 des Bereitstellens von Einstellungskriterien von vorhergehenden Einstellungen und/oder Anpassungen des Parameterwerts des zumindest einen Parameters des Magnetresonanzprotokolls können dabei zeitgleich oder in einer wie immer gearteten Reihenfolge erfolgen.

Die Einstellungskriterien umfassen Einstellungen und/oder Anpassungen des Parameterwerts des zumindest einen Parameters von früheren Magnetresonanzuntersuchungen mit dieser Magnetresonanzsequenz. Zudem umfassen die Einstellungskriterien Bewertungen zu den vorhergehenden Einstellungen und/oder Anpassungen des Parameterwerts. Die bereits vorliegenden Einstellungen und/oder Anpassungen des Parameterwerts sowie die Bewertungen der Einstellungen und/oder Anpassungen des Parameterwerts sind in einem Speichermedium und/oder einer Datenbank hinterlegt. Das Speichermedium und/oder die Datenbank sind im vorliegenden Ausführungsbeispiel von der externen Speichereinheit 27 umfasst. Zudem kann das Speichermedium und/oder die Datenbank in einer alternativen Ausgestaltung auch von der Steuerungseinheit 22 umfasst sein. Die Steuerungseinheit 22 kann mittels eines Datennetzwerks auf die in der externen Speichereinheit 27 hinterlegten Daten zugreifen.

Nach dem Bereitstellen der Rahmenbedingungen für die Einstellung und/oder Anpassung des Parameterwerts des zumindest einen Parameters des Magnetresonanzprotokolls und dem Bereitstellen der Einstellungskriterien von früheren Einstellungen und/oder Anpassungen des Parameterwerts des zumindest einen Parameters des Magnetresonanzprotokolls erfolgt anschließend in einem weiteren Verfahrensschritt 103 eine Einstellen und/oder Anpassen des Parameterwerts des zumindest einen Parameters des Magnetresonanzprotokolls. Das Einstellen und/oder das Anpassen des Parameterwerts erfolgt hierbei in Abhängigkeit von den bereitgestellten Rahmenbedingungen und den in Abhängigkeit von den bereitgestellten Einstellungskriterien.

Zur Ausführung des Verfahrensschritts des Einstellens und/oder des Anpassens des Parameterwert des zumindest einen Parameters des Magnetresonanzprotokolls für die zumindest eine Magnetresonanzsequenz wird von der Steuerungseinheit 22 ein selbstlernenden Algorithmus ausgeführt. Mittels des selbstlernenden Algorithmus erfolgt das Anpassen und/oder Einstellen des Parameterwerts des zumindest einen Parameters automatisch und/oder selbsttätig.

Der selbstlernende Algorithmus ist hierbei innerhalb der externen Speichereinheit 27 gespeichert. Zudem kann der selbstlernende Algorithmus auch innerhalb eines Speichers der Steuerungseinheit gespeichert sein.

Der selbstlernende Algorithmus basiert auf einem maschinellen Lernen, insbesondere auf einem Deep-Learning-Verfahren, bei dem Wissen aus Erfahrung generiert wird. Beim Deep-Learning-Verfahren werden künstliche neuronale Netze zu Ebenen angeordnet, die immer komplexere Merkmale verwenden, um beispielsweise den Inhalt eines Bilds zu erkennen. So könne beispielsweise große Datenbestände in Kategorien eingeteilt werden.

Der selbstlernende Algorithmus, insbesondere das Deep-Learning-Verfahren, kann dabei Muster und/oder Gesetzmäßigkeiten in Lerndaten erkennen. Die Lerndaten umfassen die in der Speichereinheit 27 hinterlegten vorhergehenden eingestellten und/oder angepassten Parameterwerte der Parameter sowie die Bewertung zu diesen eingestellten und/oder angepassten Parameterwerten. Zudem können die Lerndaten auch die mittels der eingestellten und/oder angepassten Parameterwerte der Magnetresonanzsequenz akquirierten Bilddaten umfassen, so dass weitere Kriterien, wie beispielsweise ein Bildkontrast usw. für die Erkennung von Mustern und/oder Gesetzmäßigkeiten in den Lerndaten zur Verfügung stehen.

Die bereits vorhandenen Daten zu den eingestellten und/oder angepassten Parameterwerten werden hierbei genutzt, um den selbstlernenden Algorithmus, insbesondere das maschinelle Lernen, wie beispielsweise das Deep-Learning-Verfahren, hinsichtlich der Rahmenbedingungen zu trainieren. Beispielsweise kann der selbstlernende Algorithmus, insbesondere das maschinelle Lernen, wie beispielsweise das Deep-Learning-Verfahren, hinsichtlich eines Bildkontrasts und/oder eines T1-Kontast und/oder weiteren, dem Fachmann als sinnvoll erscheinenden Rahmenbedingungen trainiert werden.

Beispielsweise reicht es für eine Einstellung und/oder Anpassung von Parameterwerten aus, wenn der Benutzer die Rahmenbedingungen vorgibt, wie beispielsweise eine Magnetresonanzuntersuchung mit einem T1-Kontrast und einer bestimmten Schichtdicke und Bildabdeckung. Alle weiteren einzustellenden und/oder anzupassenden Parameterwerte von Parametern des Magnetresonanzprotokolls werden daraufhin automatisch und/oder selbsttätig mittels des selbstlernenden Algorithmus, insbesondere des maschinellen Lernens, wie beispielsweise mittels des Deep-Learning-Verfahrens, unter Berücksichtigung der vorgegebenen und/oder bereitgestellten Rahmenbedingungen und der vorgegebenen und/oder bereitgestellten Einstellungskriterien ermittelt und eingestellt.

Zudem wird dem Verfahrensschritt des Einstellens und/oder des Anpassens des Parameterwert des zumindest einen Parameters des Magnetresonanzprotokolls kontrolliert und/oder überwacht, ob weitere Zielvorgaben eingehalten werden. Die weiteren Zielvorgaben können Sicherheitsvorgaben und/oder patientenspezifischen Zielvorgaben umfassen, wie beispielsweise eine spezifische Absorptionsrate.

Wird in dem Verfahrensschritt des Einstellens und/oder des Anpassens des Parameterwert des zumindest einen Parameters des Magnetresonanzprotokolls festgestellt, dass die Anpassung und/oder Einstellung des Parameterwerts gemäß den Vorgaben der Rahmenbedingungen und der Vorgaben der Einstellungskriterien zu einem Überschreiten einer weiteren Zielvorgabe führen würde, wird automatisch mittels des selbstlernenden Algorithmus, insbesondere mittels des maschinellen Lernens, wie beispielsweise mittels des Deep-Learning-Verfahrens, eine alternative Anpassungsstrategie gewählt. Die alternative Anpassungsstrategie umfasst eine Änderung und/oder Anpassung von mehreren Parameterwerten von weiteren Parametern des Magnetresonanzprotokolls. Zudem kann die alternative Anpassungsstrategie auch zu einer Änderung von Rahmenbedingungen führen.

Die alternative Anpassungsstrategie wird hierbei mittels des selbstlernenden Algorithmus, insbesondere des maschinellen Lernens, wie beispielsweise des Deep-Learning-Verfahrens, situationsabhängig ausgewählt. Dabei werden Parameterwerten von denjenigen Parametern des Magnetresonanzprotokolls angepasst und/oder geändert, die eine minimale Änderung der Rahmenbedingungen und/oder der Einstellungskriterien hervorrufen.

Nachdem die Parameterwerte eingestellt und/oder angepasst sind werden diese Parameterwerte gespeichert und stehen somit für eine nächste Anwendung der Magnetresonanzsequenz zur Verfügung. Die Parameterwerte werden hierbei in dem Speichermedium und/oder der Datenbank, die die bereits vorhergehenden Einstellungen und/oder Anpassungen von Parameterwerten enthalten, gespeichert. Zudem wird auch eine Bewertung nach einem Ausführen der Magnetresonanzsequenz von einem Benutzer über die Benutzerschnittstelle abgegeben und die Bewertung zusammen mit den angepassten und/oder eingestellten Parameterwerten gespeichert. Das Speichern der eingestellten und/oder angepassten Parameterwerte zusammen mit der Bewertung für die Anpassung und/oder Einstellung der Parameterwerte erfolgt in einem weiteren Verfahrensschritt 104. In dem Verfahrensschritt 104 können zusätzlich auch die mit der Magnetresonanzsequenz erfassten Bilddaten zusammen mit den eingestellten und/oder angepassten Parameterwerten gespeichert werden.

Das oben dargestellte Verfahren ist exemplarisch anhand der Einstellung und/oder Anpassung eines Parameterwerts eines Parameters des Magnetresonanzprotokolls beschrieben. Grundsätzlich kann das Verfahren für beliebig viele Parameterwerte und/oder beliebig viele Parameter des Magnetresonanzprotokolls ausgeführt werden, ohne dabei den Schutzbereich der Erfindung zu verlassen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einem Einstellen und/oder Anpassen von einem Parameterwert von zumindest einem Parameter eines Magnetresonanzprotokolls für zumindest eine Magnetresonanzsequenz mit den folgenden Verfahrensschritten:
- Auswahl des Magnetresonanzprotokolls,
- Bereitstellen von Rahmenbedingungen für die Einstellung und/oder Anpassung des Parameterwerts des zumindest einen Parameters des Magnetresonanzprotokolls,
- Bereitstellen von Einstellungskriterien von vorhergehenden Einstellungen und/oder Anpassungen des Parameterwerts des zumindest einen Parameters des Magnetresonanzprotokolls und
- Einstellen und/oder Anpassen des Parameterwerts des zumindest einen Parameters des Magnetresonanzprotokolls in Abhängigkeit von den bereitgestellten Rahmenbedingungen und in Abhängigkeit von den bereitgestellten Einstellungskriterien.

2. Verfahren nach Anspruch 1,
**dadurch gekennzeichnet, dass** das Einstellen und/oder Anpassen des Parameterwerts des zumindest einen Parameters des Magnetresonanzprotokolls automatisch und/oder selbsttätig mittels einer Steuerungseinheit (22) erfolgt, wobei die Steuerungseinheit (22) einen selbstlernenden Algorithmus aufweist.

3. Verfahren nach Anspruch 2,
**dadurch gekennzeichnet, dass** der selbstlernenden Algorithmus alle hinterlegten Einstellungskriterien für eine Anpassung und/oder Einstellung des Parameterwerts des zumindest einen Parameters des Magnetresonanzprotokolls berücksichtigt.

4. Verfahren nach einem der vorhergehenden Ansprüche,
**dadurch gekennzeichnet, dass** die Rahmenbedingungen für die Einstellung und/oder Anpassung des Parameterwerts des zumindest einen Parameters des Magnetresonanzprotokolls einen Wertebereich für den einzustellenden und/oder anzupassenden Parameterwert umfassen.

5. Verfahren nach einem der vorhergehenden Ansprüche,
**dadurch gekennzeichnet, dass** die Rahmenbedingungen für die Einstellung und/oder Anpassung des Parameterwerts des zumindest einen Parameters des Magnetresonanzprotokolls zumindest einen weiteren Parameterwert eines weiteren Parameters des Magnetresonanzprotokolls umfassen, wobei der weitere Parameterwert einen fixen Parameterwert umfasst.

6. Verfahren nach einem der vorhergehenden Ansprüche,
**dadurch gekennzeichnet, dass** die Rahmenbedingungen für die Einstellung und/oder Anpassung des Parameterwerts des zumindest einen Parameters des Magnetresonanzprotokolls zielorientierte Rahmenbedingungen umfassen.

7. Verfahren nach Anspruch 6,
**dadurch gekennzeichnet, dass** die zielorientierten Rahmenbedingungen eine Messzeit und/oder eine Bildqualität und/oder eine Schichtdicke und/oder ein Kontrastverhältnis und/oder Signal/Rauschverhältnis umfassen.

8. Verfahren nach einem der vorhergehenden Ansprüche,
**dadurch gekennzeichnet, dass** die Rahmenbedingungen für die Einstellung und/oder Anpassung des Parameterwerts des zumindest einen Parameters des Magnetresonanzprotokolls benutzerabhängig sind.

9. Verfahren nach einem der vorhergehenden Ansprüche,
**dadurch gekennzeichnet, dass** die Einstellungskriterien bereits erfolgte Einstellungen und/oder Anpassungen des Parameterwerts des zumindest einen Parameters des Magnetresonanzprotokolls und eine Bewertung der bereits erfolgten Einstellungen und/oder Anpassungen des Parameterwerts des zumindest einen Parameters umfassen.

10. Verfahren nach einem der vorhergehenden Ansprüche,
**dadurch gekennzeichnet, dass** bei einem Überschreiten der Rahmenbedingungen und/oder von weiteren Zielvorgaben während der Anpassung und/oder Einstellung des Parameterwerts des zumindest einen Parameters des Magnetresonanzprotokolls automatisch eine alternative Anpassungsstrategie gewählt wird, bei der mehrere Parameterwerte von weiteren Parametern des Magnetresonanzprotokolls angepasst und/oder geändert werden.

11. Verfahren nach Anspruch 10,
**dadurch gekennzeichnet, dass** die Anpassungsstrategie situationsabhängig ausgewählt wird.

12. Verfahren nach einem der Ansprüche 10 bis 11,
**dadurch gekennzeichnet, dass** die Anpassungsstrategie diejenigen Parameterwerte von den weiteren Parametern des Magnetresonanzprotokolls anpasst und/oder ändert, die eine minimale Änderung der Rahmenbedingungen und/oder der Einstellungskriterien hervorrufen.

13. Verfahren nach einem der vorhergehenden Ansprüche,
**dadurch gekennzeichnet, dass** der eingestellte und/oder angepasste Parameterwert des zumindest einen Parameters des Magnetresonanzprotokolls gespeichert wird.

14. Verfahren nach einem der vorhergehenden Ansprüche,
**dadurch gekennzeichnet, dass** der eingestellte und/oder angepasste Parameterwert des zumindest einen Parameters des Magnetresonanzprotokolls bewertet wird und die Bewertung zusammen mit dem eingestellten und/oder angepassten Parameterwert des zumindest einen Parameters des Magnetresonanzprotokolls gespeichert wird.

15. Magnetresonanzvorrichtung mit einer Steuerungseinheit (22) und einer Benutzerschnittstelle (23), wobei die Magnetresonanzvorrichtung zu einer Durchführung eines Verfahrens zu einem Einstellen und/oder Anpassen von einem Parameterwert zumindest eines Parameters eines Magnetresonanzprotokolls für zumindest Magnetresonanzsequenz ausgelegt ist.

16. Computerprogrammprodukt, welches ein Programm umfasst und direkt in einen Speicher einer programmierbaren Steuerungseinheit (22) ladbar ist, mit Programmmitteln, um ein Verfahren zu einem Einstellen und/oder Anpassen von einen Parameterwert zumindest eines Parameters eines Magnetresonanzprotokolls für zumindest Magnetresonanzsequenz nach einem der Ansprüche 1 bis 14 auszuführen, wenn das Programm in der Steuerungseinheit (22) ausgeführt wird.

17. Elektronisch lesbarer Datenträger mit darauf gespeicherten elektronisch lesbaren Steuerinformationen, welche derart ausgestaltet sind, dass sie bei Verwendung der Steuerinformationen in einer Steuerungseinheit (22) ein Verfahren zu einem Einstellen und/oder Anpassen von einem Parameterwert zumindest eines Parameters eines Magnetresonanzprotokolls für zumindest Magnetresonanzsequenz nach einem der Ansprüche 1 bis 14 durchführen.
